# EUROPEAN PATENT SPECIFICATION

(11) **EP 2 415 488 B1**
(45) Date of publication and mention of the grant of the patent: **07.03.2018**
(21) Application number: 11176575.6
(22) Date of filing: 04.08.2011
(51) Int. Cl.: A61L 17/04, A61L 17/14

(54) **Thread, especially surgical thread, an implant comprising the thread and also a process for producing the thread and the implant**
Faden, insbesondere ein chirurgischer Faden, Implantat mit dem Faden sowie ein Verfahren zur Herstellung des Fadens und des Implantats
Fil, spécialement un fil chirurgical, implant comportant le fil et également un procédé pour produire le fil et l'implant

(30) Priority: 06.08.2010 DE 102010034471
(43) Date of publication of application: 08.02.2012
(73) Proprietor: Aesculap AG, 78532 Tuttlingen (DE); B. Braun Surgical S.A., 08191 Rubi Barcelona (ES)
(72) Inventor: Gonzalez Calero, Marta, ES-08191 Rubi (ES); Odermatt, Erich, CH-8200 Schaffhausen (CH)
(74) Representative: Patentanwälte Ruff, Wilhelm, Beier, Dauster & Partner mbB

(56) References cited:
- EP-A1- 1 543 848
- EP-B1- 1 897 500
- WO-A1-2004/045663
- WO-A1-2010/127874
- US-A1- 2002 156 150
- US-A1- 2008 051 834

## Description

The present invention relates to a thread, especially a surgical thread, an implant comprising the thread and also a production process for the thread and the implant.

Ever since surgical suture was first used, the suture material has undergone constant further development to the present day. The natural materials originally used have come to be largely replaced by synthetic suture materials. Depending on the field of surgery, the suture materials used are resorbable or nonresorbable or else optionally partially resorbable.

While nonresorbable suture materials are typically used for vascular, especially cardiovascular, and orthopedic applications, resorbable suture materials are mainly used in gastrointestinal, gynecological and plastic surgery, especially for skin stretching, and for approximation of soft tissue.

Nonresorbable suture materials suitable in principle can consist of ultrahigh molecular weight polyethylene (UHMWPE). Suture materials of this type are notable for very high tensile strengths. A disadvantage is the smooth/slippery surface of suture materials made of UHMWPE. This slipperiness can impair the knot security of a suture and thus endanger wound closure. To nonetheless be able to ensure secure wound closure, the surgeon frequently has to place two or more knots, in some instances even more than six knots, on top of each other. This means an increased introduction of foreign material into the patient, heightening the risk of inflammatory reactions and tissue erosions. Apart from that, the superposition of several knots to ensure adequate knot security is an inconvenient procedure for the surgeon and also susceptible to error in principle. When knots are placed too loosely, this can cause wound dehiscence. When the knots are placed too firmly, by contrast, outcomes may be cosmetically unsatisfactory owing to tissue necrosis.

EP 1 543 848 A1 describes technical teaching which goes in another direction. The suture material proposed has a core-sheath construction where the core includes a bioabsorbable polymer. The sheath is a braid and comprises a nonabsorbable yarn and a bioabsorbable yarn. The suture material thus consists predominantly of resorbable material, but this is in turn disadvantageous for its tensile strength and hence mechanical stability.

EP 1 897 500 B1 discloses a barbed surgical suture comprising an elongated body comprising polyethylene, an effective amount of a bioactive agent and optionally a coating comprising a degradable polymer.

EP 1 543 848 A1 discloses a coating suture comprising a suture body having a core-sheath structure, wherein the core consists of polydioxanone yarns and the sheath consists of ultrahigh molecular weight polyethylene yarns and polydioxanone yarns and the coating is made of a copolymer of caprolactone/glycolide.

US 2008/0051834 A1 discloses a suture comprising a braided jacket comprising a plurality of yarns formed of ultrahigh molecular weight polyethylene and in particular of yarns formed of polyester, wherein the suture is coated with collagen.

The problem addressed by the present invention is therefore that of providing a thread which is improved in respect of wound closure and especially the security of wound closure. In contradistinction to existing threads of the type in question, the thread proposed herein is especially notable in that less foreign material has to be introduced into the body of a patient to ensure secure wound closure.
This problem is solved according to the present invention by a thread, especially a medical or surgical thread according to claim 1, which comprises an elongate thread body and a coating surrounding the thread body at least partially, preferably completely, i.e. all-over. The thread body consists of polyethylene. The coating consists of a resorbable material and optionally of additives.
According to the present invention, it is particularly favorable for the thread body to consist of polyethylene.
The polyethylene is an ultrahigh molecular weight polyethylene (UHMWPE). This is a particularly advantageous way of providing a medically optimal mechanical stability, especially linear tensile strength, for the thread.
To further improve the mechanical stability of the thread, it may be provided according to the present invention that the polyethylene is present in a crosslinked state, especially in a chemically and/or physically crosslinked state. For example, the polyethylene can be present in a crosslinked state as a consequence of a peroxide treatment. Physical crosslinking of polyethylene can be effected for example using irradiation, especially ionizing irradiation. For example, the polyethylene can be present in a crosslinked state as a result of a treatment with γ-rays, β-rays, x-rays, ultraviolet rays, neutron beam rays, proton beam rays and/or electron beam rays.

In a particularly preferred embodiment, the polyethylene is a crosslinked ultrahigh molecular weight polyethylene (UHMWPE). Concerning suitable methods of crosslinking, the preceding embodiment is referenced in its entirety.

The polyethylene is preferably an ultrahigh molecular weight polyethylene (UHMWPE) having an average molecular weight between 10⁴ and 10⁷ g/mol, especially 10⁵ and 10⁶ g/mol.

The thread body in a particularly advantageous embodiment is a multifil, especially braided or twisted, thread body, especially a multifilament yarn. In this way, the advantages of a multifil thread such as flexibility and tying properties for example can also be realized for the thread of the present invention. The thread body is preferably a braided thread body. It can be especially provided according to the present invention for the thread body to be configured as a braid with a core. In specific embodiments, individual filaments of a multifil thread body can be bonded, especially melted, together.

In one possible embodiment, the thread body has a monofil configuration.

It can further be provided according to the present invention that the thread body is present as a pseudo monofilament.

The coating of the thread is a nontextile coating. Such a coating provides for example a distinct reduction in the capillarity and the attendant potential infection risk for an invention thread having a multifil thread body. A nontextile coating also has the advantage that it can be applied to the thread body, or produced together with the thread body, using relatively simple techniques. Appropriate techniques are more particularly described hereinbelow.

The coating or to be more precise the coating surface has a certain roughness in a preferred embodiment. More particularly, fine hair cracks or fissures can be formed on the surface of the coating in the event of the thread being subjected to a load, increasing the thread's coefficient of friction. An increased coefficient of friction for the thread means increased knot security and hence a secure wound closure. Improved friction on the thread more particularly means that fewer knots are needed to bring about secure wound closure. This means reduced material requirements and more particularly a lower input of foreign material into the body of a patient.
The resorbable material of the coating may in principle be a single resorbable material or alternatively a mixture of different resorbable materials. Suitable resorbable materials are more particularly described hereinbelow:
The resorbable material is a polymer, preferably a synthetic polymer. The polymer may be a copolymer in particular. For example, the resorbable material may be present in the form of a random copolymer and/or block copolymer such as a di- and/or triblock copolymer for example.

The resorbable material is a polyhydroxyalkanoate . The resorbable material is preferably a polyhydroxyalkanoate with two or more different hydroxyalkanoate units.

The resorbable material is alternatively a polymer selected from the group consisting of polyglycolide, polylactide, poly-3-hydroxybutyrate, poly-4-hydroxybutyrate, polytrimethylene carbonate, poly-para-dioxanone, poly-ε-caprolactone, copolymers thereof and mixtures, especially blends, thereof.

The resorbable material is more preferably a copolymer based on glycolide and lactide, preferably in a weight ratio ranging from 9:1 to 1:9 and especially from 7:3 to 3:7. Further preferred copolymers comprise ε-caprolactone, trimethylene carbonate and a glycolide or glycolide and ε-caprolactone. An especially preferred copolymer is a terpolymer, in particular triblock terpolymer, made of glycolide, trimethylene carbonate and ε-caprolactone. Such a terpolymer is commercially available under the trademark Monosyn®.

In principle, the coating on the thread can consist exclusively of the resorbable material.

However, it may be preferable according to the present invention for the coating to consist of the resorbable material and the additives. The additives make it possible for the thread to be endowed with medically, especially therapeutically, advantageous properties.

The expression "additives" for the purposes of the present invention shall comprise not only a single additive but also a mixture of two or more different additives. The additives may further be polymeric additives or nonpolymeric, i.e., low molecular weight, additives.

Preferred additives are medical/pharmaceutical actives. Advantageous additives can be selected for example from the group consisting of antimicrobial, especially antibiotic, actives, disinfecting actives, active promoters of wound healing, anti-inflammatory actives, analgesic actives, cellular growth factors, cellular differentiation factors, cellular recruitment factors, cellular adhesion factors, derivatives thereof, salts thereof and mixtures thereof.

Preferred antimicrobial actives can be organic compounds and/or metals, especially metal salts such as metal oxides for example. Examples of antimicrobially active organic compounds can be selected from the group consisting of polyhexamethylenebiguanide, chlorhexidine, derivatives thereof, salts thereof and mixtures thereof. Advantageous antimicrobially active metals and metal salts can be selected from the group consisting of copper, silver, gold, salts, especially oxides, thereof and mixtures thereof.

In a particularly preferred embodiment, the optional additives of the present invention include salts, especially organic salts, preferably fatty acid salts and more preferably alkali metal fatty acid salts and/or alkaline earth metal fatty acid salts. For example, the additives may include magnesium and/or calcium fatty acid salts, especially magnesium and/or calcium stearate.

In a more advanced embodiment, the additives, preferably in the form of fatty acid salts, account for a proportion between 0.5 wt. % and 5 wt.%, especially 1.5 wt.% and 2.5 wt.% and preferably 1 wt.% and 2 wt.%, based on the total weight of the thread.

The coating itself preferably accounts for a proportion between 1 wt.% and 9 wt.%, especially 2 wt.% and 6 wt.%, preferably 3 wt.% and 6 wt.% and more preferably 2.5 wt.% and 4.5 wt.%, based on the total weight of the thread.

In a further embodiment, the coating has a layer thickness between 1 µm and 100 µm, especially 3 µm and 80 µm and preferably 5 µm and 50 µm.

The coating is preferably compact and, more particularly, sealing.

The thread further preferably has a linear tensile strength between 10 N and 250 N, especially 20 N and 210 N and more preferably 30 N and 190 N. Linear tensile strength for the purposes of the present invention is the force in newtons [N] needed to break the thread, as measured on the straightened thread.

In a further advantageous embodiment, the thread has a knot breaking strength between 5 N and 140 N, especially 15 N and 120 N and preferably 20 N and 100 N. Knot breaking strength for the purposes of the present invention is the force in newtons [N] needed to break the knotted thread, as measured in the knot of the thread.

The flexibility of the thread is preferably between 1 mN and 50 mN, especially 3 mN and 40 mN and preferably 5 mN and 30 mN.

In a preferred embodiment, the thread has a core-sheath construction where the core is formed by the thread body and the sheath is formed by the coating. More particularly, the thread can be configured as a coextrusion thread or sheath extrusion thread.

The thread can be of monofil or multifil configuration. The thread is preferably a pseudo monofil thread. In other words, it can be preferable for the thread body itself to be present as a multifilament. The thread body can be present as a braided structure in particular. The thread body is preferably formed as a braid with a core.

In a further advantageous embodiment, the thread is present as a sterilized and preferably end-itemed, especially cut-to-length, thread.

In a particularly preferred embodiment, the thread is configured as surgical suture material.

In an advanced embodiment, the thread is attached to one or more, especially two, surgical needles.

A further aspect of the present invention relates to an implant, especially a medical/surgical implant, comprising at least one, especially one, thread, especially one medical/surgical thread, having an elongate, preferably braided, thread body and a coating surrounding the thread body at least partially, preferably completely, wherein the thread body consists of polyethylene and the coating consists of a resorbable material and optionally additives.

The implant is preferably a textile implant. More particularly, the implant may be executed as textile mesh, for example as hernia mesh, urinary incontinence mesh or prolapse mesh. In addition, the implant of the present invention may in principle also comprise other textile implants such as, for example, vascular prostheses, stents, stent linings or the like.

However, it is particularly preferable for the implant to be configured as a surgical suture material.

Concerning further features and advantages, especially in relation to the thread, the thread body and the coating, express reference is made to the description heretofore.

The present invention further also comprises a process for producing a thread, especially a medical/surgical thread, or an implant, especially a medical/surgical implant, wherein an elongate, preferably braided, thread body comprising polyethylene is coated with a resorbable material and optionally additives.

In principle, the coating of the thread body may only be partial. Preferably, however, the thread body is coated completely, i.e., all-over, with the resorbable material and optionally the additives.

In an advantageous embodiment, the coating can be effected using a core-sheath extrusion. This is generally accomplished via a coextrusion of the thread body and of the coating. In the process, a core-sheath construction can be realized. For example, a bicomponent extrusion can be carried out to produce the thread/implant.

In an alternative embodiment, the thread body is coated with the resorbable material and optionally the additives using a sheathing extrusion. In this embodiment, the thread body can be used as monofilament or multifilament, especially multifilament yarn. When a multifil thread body is used, this can be used to produce pseudo monofil threads having the properties described above.

In a further embodiment, the coating of the thread body is effected using a soaking, wetting, dipping, spraying, brushing and/or calendering technique. Depending on the particular coating technique used, it is advantageous to use the resorbable material in the form of a dispersion, suspension, solution or melt.

The above-described techniques for coating the thread body are relatively simple and, more particularly, economical to carry out and moreover permit not only a partial coating but also a complete, i.e., all-over, coating of the thread body.

As mentioned, the present invention provides the option of additionally coating the thread body with additives. These additives for coating the thread body may already be present in the resorbable material. For this, dispersions, suspensions, solutions or melts of the resorbable material can be provided with the additives for example. This approach has the advantage of allowing a uniform/homogeneous distribution of the additives in the resorbable material and so with particular advantage a similarly uniform/homogeneous distribution of the additives in the coating of the final thread is obtainable.

Alternatively, the optional additives according to the present invention can also be introduced into the coating only in a subsequent treatment step of the thread.

Concerning further features and advantages of the process, especially in relation to the thread, the thread body and the coating, express reference is made to the description heretofore.

Finally, it is disclosed the use of a thread comprising an elongate, preferably braided, thread body and a coating surrounding the thread body at least partially and preferably completely, wherein the thread body comprises polyethylene and the coating consists of a resorbable material and optionally additives, for producing an implant, especially a surgical implant, preferably a surgical suture material.

To avoid unnecessary repetition, concerning further features and advantages of the thread, especially in relation to its thread body and coating, the preceding description is likewise referenced in its entirety.

At this point, the advantages of the present invention will be summarized once more as follows:
The thread of the present invention is particularly advantageous in that, firstly, its material constitution of its thread body provides it with a very high basic mechanical stability which facilitates especially the passage through tissue and the placing of knots. This supports the achievement of secure wound closure.

Secondly, the coating on the thread provides it with improved friction properties which lead to a distinct improvement in knot security, especially knot breaking strength. More particularly, the improved friction of the thread requires fewer knots to be placed to produce a secure wound closure which is riskless for the patient. As a result, less thread material and hence less foreign material is introduced into the body of the patient. As a result, the risk of undesirable secondary reactions such as rejection reactions or tissue erosions, which can be attributable to the shear volume of suture material knots, is distinctly minimized.

The fact that fewer knots have to be placed to ensure secure wound closure significantly simplifies the handling of the thread for the physician/surgeon involved. In addition, the propensity to mistakes in placing the knots and hence in bringing about wound closure can be distinctly reduced in this way.

A further advantage of the thread is that, when the thread body is multifil, especially braided, the coating provided according to the invention ensures that the thread shows distinctly reduced capillarity. This makes it possible to take advantage of the inherent virtues of a multifil thread, such as flexibility and tying properties for example, while at the same time incurring a distinctly reduced risk of capillary-based infections.

Further features and advantages of the invention become apparent from the ensuing description of preferred embodiments with reference to examples in conjunction with the features of dependent claims. Individual features of the invention may here be actualized each on its own or in combination with each or one another. The embodiments described serve for elucidation and better understanding of the invention and are not in any way to be understood as restricting.

### Examples

### 1. Material

The material used was a UHMWPE fiber having an approximate molecular weight of 2.5 × 10⁶ g/mol (Dyneema®) .

### 2. Coating

The fibers to be coated were led from one spool over a further spool to endow the fibers with the desired speed and tension. The fibers were subsequently routed into a coating bath and led over a further spool through a heating duct. The length of the fibers within the heating duct was adjustable via mobile spools, and so parameters such as, for example, fiber tension and residence time of the fibers in the heating duct were also adjustable. The dried fibers were led out of the heating duct and wound up on a further spool.

The fibers thus coated had a coating proportion of about 2% by weight, based on the total weight of the coated fiber.

The fibers were coated using the following solutions:
1^{st} solution containing a copolymer based on glycolide (54 wt.%) and L-lactide (46 wt.%) and also calcium stearate (ratio of copolymer to calcium stearate: 2:1), and
2^{nd} solution containing a copolymer based on ε-caprolactone (60 wt.%), trimethylene carbonate (30 wt.%) and glycolide (10 wt.%) with or without calcium stearate.

Uncoated Dyneema® threads were used as comparative fibers.

### 3. Knot slide

To measure the roughness of the fibers coated with the 2nd solution, the fibers were clamped into a device for measuring knot slide. The lower free ends of the fibers each had a weight of 200 g suspended from them. The measurement was based on the following parameters:
change of direction speed: 500 mm/min
test path: 150 mm, of which the first 50 mm were not measured, the measurement only being based on the subsequent 75 mm.

The measurements showed that friction was more than 50% lower for the coated fibers versus uncoated fibers.

### 4. Knot security factor

A surgical knot (2 = 1) was made in a cylinder having a circumference of about 20 cm. The knot ears were cut at a distance of 8 to 10 mm. The fibers were cut at opposite regions of the knot. The ends obtained were subsequently tightened in dynamometer clamps. The dynamometer pulled at a constant speed of 100 mm/min. The fibers had to break open in the knot in 10 test runs (knot security factor = 0). If the knot slipped, an additional throw had to be added to the surgical knot. In this case, the knot geometry was then 2 = 1 = 1. The number of throws added is the knot security factor. For example, a knot geometry of 2 = 1 = 1 = 1 corresponds to a knot security factor of 2.

The uncoated fibers had a knot security factor of more than 6, while the coated fibers had a knot security factor of less than 4. This result illustrates that the coated fibers needed fewer knots to provide correct wound closure.

### 5. Capillarity test

This test was used to investigate the capillarity of braided UIIMWPE threads produced from coated or uncoated UHMWPE fibers. For this, the threads were vertically dipped into an aqueous solution of methylene blue (0.1% w/v). The lower ends of the threads were fixed with a weight of 2 g. The capillarity of the threads caused the solution to rise up in the threads.

In general, a thread has a medically relevant capillarity when the colored length is > 1 cm after one hour.

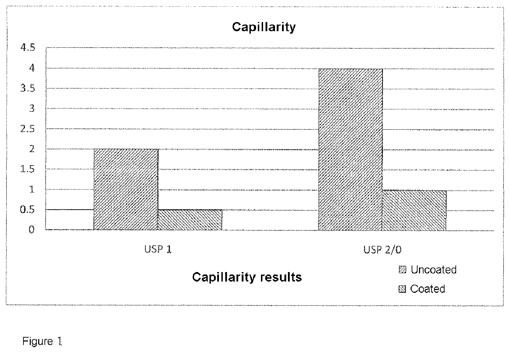

The uncoated threads had a colored length of 1.8 cm and 3.7 cm (USP1 and USP2/0 respectively) after one hour. By contrast, the coated threads had a colored length of 0.4 cm (USP1) and 0.8 cm (USP2-/0). The results are graphed in figure 1 for clarity.

## Claims

1. Thread, especially surgical thread, comprising an elongate thread body and a coating surrounding the thread body at least partially, **characterized in that** the thread body consists of polyethylene and the coating consists of a resorbable material and optionally additives,wherein the polyethylene is an ultrahigh molecular weight polyethylene, the resorbable material is a polyhydroxyalkanoate or a polymer selected from the group consisting of polyglycolide, polylactide, poly-3-hydroxybutyrate, poly-4-hydroxybutyrate, polytrimethylene carbonate, poly-para-dioxanone, poly-s-caprolactone, copolymers thereof and mixtures thereof and the coating is a non-textile coating.

2. Thread according to claim 1, **characterized in that** the polyethylene is a crosslinked ultrahigh molecular weight polyethylene.

3. Thread according to claim 1 or 2, **characterized in that** the resorbable material is a polyhydroxyalkanoate with two or more different hydroxyalkanoate units.

4. Thread according to any preceding claim, **characterized in that** the resorbable material is a copolymer based on glycolide and lactide, preferably in a weight ratio ranging from 9:1 to 1:9, especially from 7:3 to 3:7.

5. Thread according to any preceding claim, **characterized in that** the additives, especially at least partly, are fatty acid salts, especially alkali metal and/or alkaline earth metal fatty acid salts, preferably magnesium and/or calcium fatty acid salts, especially magnesium and/or calcium stearate.

6. Thread according to any preceding claim, **characterized in that** the additives, preferably in the form of fatty acid salts, account for a proportion between 0.5 and 5 wt.%, especially 1.5 and 2.5 wt.% and preferably 1 and 2 wt.%, based on the total weight of the thread.

7. Thread according to any preceding claim, **characterized in that** the coating accounts for a proportion between 1 and 9 wt.%, especially 2 and 6 wt.% and preferably 2.5 and 4.5 wt.%, based on the total weight of the thread.

8. Thread according to any preceding claim, **characterized in that** the coating has a layer thickness between 1 and 100 µm, especially 3 and 80 µm and preferably 5 and 50 µm.

9. Implant, preferably in the form of a surgical suture material, comprising a thread according to any preceding claim.

10. Process for producing a thread according to any one of claims 1 to 8 or an implant according to claim9, wherein an elongate thread body consisting of polyethylene is at least partially coated with a resorbable material and optionally additives, wherein the polyethylene is an ultrahigh molecular weight polyethylene and the resorbable material is a polyhydroxyalkanoate or a polymer selected from the group consisting of polyglycolide, polylactide, poly-3-hydroxybutyrate, poly-4-hydroxybutyrate, polytrimethylene carbonate, poly-para-dioxanone, poly-s-caprolactone, copolymers thereof and mixtures thereof.

## Patentansprüche

1. Faden, insbesondere chirurgischer Faden, umfassend einen länglichen Fadenkörper und eine den Fadenkörper zumindest teilweise umgebende Beschichtung,
**dadurch gekennzeichnet, dass**
der Fadenkörper aus Polyethylen gebildet ist und die Beschichtung aus einem resorbierbaren Material und gegebenenfalls Additiven gebildet ist, wobei es sich bei dem Polyethylen um ultra-hochmolekulares Polyethylen handelt, es sich bei dem resorbierbaren Material um ein Polyhydroxyalkanoat handelt oder um ein Polymer, das ausgewählt ist aus der Gruppe bestehend aus Polyglykolid, Polylactid, Poly-3-Hydroxybutyrat, Poly-4-Hydroxybutyrat, Poly-Trimethylencarbonat, Poly-para-Dioxanon, Poly-ε-Caprolacton, Copolymere davon und Mischungen davon und es sich bei der Beschichtung um eine nichttextile Beschichtung handelt.

2. Faden nach Anspruch 1, **dadurch gekennzeichnet, dass** es sich bei dem Polyethylen um ein vernetztes ultra-hochmolekulares Polyethylen (UHMWPE) handelt.

3. Faden nach Anspruch 1 oder 2, **dadurch gekennzeichnet, dass** es sich bei dem resorbierbaren Material um ein Polyhydroxyalkanoat mit zwei oder mehr verschiedenen Hydroxyalkanoateinheiten handelt.

4. Faden nach einem der vorhergehenden Ansprüche, **dadurch gekennzeichnet, dass** es sich bei dem resorbierbaren Material um ein Copolymer, umfassend Glykolid und Lactid, vorzugsweise in einem Gewichtsverhältnis von 9:1 bis 1:9, insbesondere 7:3 bis 3:7, handelt.

5. Faden nach einem der vorhergehenden Ansprüche, **dadurch gekennzeichnet, dass** es sich bei den Additiven, insbesondere zumindest teilweise, um Fettsäuresalze, insbesondere um Alkalimetall- und/oder Erdalkalimetallfettsäuresalze, vorzugsweise um Magnesium- und/oder Calciumfettsäuresalze, insbesondere um Magnesium- und/oder Calciumstearat, handelt.

6. Faden nach einem der vorhergehenden Ansprüche, **dadurch gekennzeichnet, dass** die Additive, vorzugsweise in Form von Fettsäuresalzen, einen Anteil zwischen 0,5 und 5 Gew.-%, insbesondere 1,5 und 2,5 Gew.-%, vorzugsweise 1 und 2 Gew.-%, bezogen auf das Gesamtgewicht des Fadens, aufweisen.

7. Faden nach einem der vorhergehenden Ansprüche, **dadurch gekennzeichnet, dass** die Beschichtung einen Anteil zwischen 1 und 9 Gew.-%, insbesondere 2 und 6 Gew.-%, vorzugsweise 2,5 und 4,5 Gew.-%, bezogen auf das Gesamtgewicht des Fadens, aufweist.

8. Faden nach einem der vorhergehenden Ansprüche, **dadurch gekennzeichnet, dass** die Beschichtung eine Schichtdicke zwischen 1 und 100 µm, insbesondere 3 und 80 µm, vorzugsweise 5 und 50 µm, aufweist.

9. Implantat, vorzugsweise in Form eines chirurgischen Nahtmaterials, umfassend einen Faden nach einem der vorhergehenden Ansprüche.

10. Verfahren zur Herstellung eines Fadens nach einem der Ansprüche 1 bis 8 oder eines Implantats nach Anspruch 9, wobei ein länglicher Fadenkörper, der aus Polyethylen gebildet ist, zumindest teilweise mit einem resorbierbaren Material und gegebenenfalls Additiven beschichtet wird, wobei es sich bei dem Polyethylen um ein ultra-hochmolekulares Polyethylen handelt und es sich bei dem resorbierbaren Material um ein Polyhydroxyalkanoat handelt oder um ein Polymer, das ausgewählt ist aus der Gruppe bestehend aus Polyglykolid, Polylactid, Poly-3-Hydroxybutyrat, Poly-4-Hydroxybutyrat, Poly-Trimethylencarbonat, Poly-para-Dioxanon, Poly-ε-Caprolacton, Copolymere davon und Mischungen davon.

## Revendications

1. Fil, en particulier fil chirurgical, comprenant un corps de fil allongé et un revêtement entourant le corps de fil au moins en partie, **caractérisé en ce que** le corps de fil se compose de polyéthylène et le revêtement se compose d'un matériau résorbable et optionnellement d'additifs, dans lequel le polyéthylène est un polyéthylène à ultra-haute masse moléculaire, le matériau résorbable est un polyhydroxyalcanoate ou un polymère sélectionné dans le groupe constitué du polyglycolide, du polylactide, du poly-3-hydroxybutyrate, du poly-4-hydroxybutyrate, du carbonate de polytriméthylène, de la poly-para-dioxanone, de la poly-ε-caprolactone, de copolymères de ceux-ci et de mélanges de ceux-ci, et le revêtement est un revêtement non textile.

2. Fil selon la revendication 1, **caractérisé en ce que** le polyéthylène est un polyéthylène à ultra-haute masse moléculaire réticulé.

3. Fil selon la revendication 1 ou 2, **caractérisé en ce que** le matériau résorbable est un polyhydroxyalcanoate comportant deux ou plusieurs différentes unités hydroxyalcanoate.

4. Fil selon l'une quelconque des revendications précédentes, **caractérisé en ce que** le matériau résorbable est un copolymère à base de glycolide et de lactide, préférablement selon un rapport pondéral de 9:1 à 1:9, en particulier de 7:3 à 3:7.

5. Fil selon l'une quelconque des revendications précédentes, **caractérisé en ce que** les additifs, en particulier au moins en partie, sont des sels d'acides gras, en particulier des sels d'acides gras de métaux alcalins et/ou de métaux alcalinoterreux, préférablement des sels d'acides gras de magnésium et/ou de calcium, en particulier le stéarate de magnésium et/ou de calcium.

6. Fil selon l'une quelconque des revendications précédentes, **caractérisé en ce que** les additifs, préférablement sous la forme de sels d'acides gras, représentent une proportion de 0,5 à 5 % en poids, en particulier de 1,5 à 2,5 % en poids, et préférablement de 1 à 2 % en poids, relativement au poids total du fil.

7. Fil selon l'une quelconque des revendications précédentes, **caractérisé en ce que** le revêtement représente une proportion de 1 à 9 % en poids, en particulier de 2 à 6 % en poids, et préférablement de 2,5 à 4,5 % en poids, relativement au poids total du fil.

8. Fil selon l'une quelconque des revendications précédentes, **caractérisé en ce que** le revêtement a une épaisseur de couche de 1 à 100 µm, en particulier de 3 à 80 µm, et préférablement de 5 à 50 µm.

9. Implant, préférablement sous la forme d'un matériau de suture chirurgicale, comprenant un fil selon l'une quelconque des revendications précédentes.

10. Procédé de fabrication d'un fil selon l'une quelconque des revendications 1 à 8 ou d'un implant selon la revendication 9, dans lequel un corps de fil allongé constitué de polyéthylène est au moins en partie revêtu d'un matériau résorbable et éventuellement d'additifs, dans lequel le polyéthylène est un polyéthylène à ultra-haute masse moléculaire et le matériau résorbable est un polyhydroxyalcanoate ou un polymère sélectionné dans le groupe constitué du polyglycolide, du polylactide, du poly-3-hydroxybutyrate, du poly-4-hydroxybutyrate, du carbonate de polytriméthylène, de la poly-para-dioxanone, de la poly-ε-caprolactone, de copolymères de ceux-ci et de mélanges de ceux-ci.
